# EUROPEAN PATENT APPLICATION

(11) **EP 2 815 733 A1**
(43) Date of publication of application: **24.12.2014**
(21) Application number: 13172533.5
(22) Date of filing: 18.06.2013
(51) Int. Cl.: A61F 13/62, A44B 18/00, B29C 43/22, D04H 13/00

(54) **Web Comprising A Fastening Material**

(71) Applicant: 3M Innovative Properties Company, Saint Paul, MN 55133-3427 (US)
(72) Inventor: Peiffer, Andreas, 41470 Neuss (DE)
(74) Representative: Aleandri-Hachgenei, Lorraine E.

(57) **Abstract**

The present invention relates to a web comprising a fastening material, to a method of manufacturing such a web, to a sonotrode adapted to bond a fastening material to a nonwoven material and to the use of such a sonotrode.

## Description

The present invention relates to a web comprising a fastening material, to a method of manufacturing such a web, to a sonotrode adapted to bond a fastening material to a non-woven material and to the use of such a sonotrode.

Webs comprising a fastening material are used in various applications, in particular in the personal care industry, e.g., as fasteners for diapers and the like. Since such fastening materials typically consist of a relatively rigid plastic material, the surface of such materials are rather uncomfortable with regard to haptics or even irritating the skin which may come into contact with said materials. Therefore, the backside of such fastening materials, if used in personal care applications, is typically covered with a layer of a softer, more comfortable and less irritating material such as, e.g., a non-woven.

US 2006/0265867 discloses an apparatus and a method of temporarily coupling a hook material to a fibrous non-loop material by introducing the hook material to the non-loop material and ultrasonically vibrating at least one of the materials to cause temporary engagement between the two materials.

JP 2011/015796 discloses a fastening tape to be used for fixing a front body to a rear body of a diaper, capable of being easily attached and having a hook member hardly removable from a base material. The fastening tape is used for fixing the front body to the rear body of the diaper. The fastening tape includes the base material made of a nonwoven fabric, and the hook member of a mechanical fastener to be adhered to a part of the surface of the base material. A surface fused part, where fiber webs on the front surface are heat-sealed to one another, is formed on the surface of the base material corresponding to the adhered region of the hook member, and the hook member is adhered to the surface fused part.

One simple and cost efficient way to bond said cover layer to a fastening material is welding, in particular ultrasound welding. By means of a sonotrode the material is welded to, e.g., a non-woven at specific bonding spots. However, due to the heat generated during welding and due to the fact that the sonotrode has a given size, several mechanical fasteners of the fastening material, which are present at the bonding spot, are being destroyed. Accordingly, the fastening material, once bonded to the layer of non-woven, has less and/or damaged mechanical fasteners which negatively affects the performance of the fastening material.

It is therefore an object of the present invention to provide an improved method of bonding a fastening material to another material as well as to provide an improved web which overcomes the above-mentioned disadvantage. This object is achieved by a web according to claim 1 and a method according to claim 8. The dependent claims refer to preferred embodiments.

The present invention relates to a web comprising a first layer of a first material and a second layer of a fastening material. The second layer has first and second major surfaces and is provided with, preferably multiple, mechanical fasteners. The mechanical fasteners may have stems on the first major surface. Furthermore, the mechanical fasteners may have caps to form hooks. The second major surface of the second material faces the first layer and the second layer is bonded to the first layer at regions between adjacent mechanical fasteners and the second layer is not bonded to the first layer at regions below the mechanical fasteners. In other words, the fastening material below a mechanical fastener is preferably not bonded to the first layer at all. Rather, the bonding spots or regions are confined in such a manner that essentially no mechanical fasteners and, more preferably, only very few and, preferably no mechanical fasteners are provided directly above any bonding spots or regions.

Mechanical fasteners as used in the invention may comprise a stem. Also, mechanical fastening may comprise an enlarged head such as a cap with or without a stem. In the latter case, the cap may be directly bonded to a substrate without a stem. Furthermore, fastening elements may comprise hooks.

Accordingly, the present invention is based on the idea to only bond the fastening material to the first material at bonding spots or regions which preferably lie entirely between adjacent mechanical fasteners. Thus, the bonding does not negatively affect the mechanical fasteners resulting in a web whose material has an improved performance.

In the context of the present invention, "bonding" includes discrete and possibly discontinuous bond lines, which are spaced from each other.

While the second layer may be bonded to the first layer by welding, preferably by ultrasound welding or thermo bonding, other bonding methods, such as laser welding, may also be conceivable.

It is further preferred that the first material is a non-woven.

Preferably, the fastening material is provided with a regular pattern of mechanical fasteners, wherein the second layer is bonded to the first layer along lines extending between adjacent mechanical fasteners. Preferably, the web defines a longitudinal and a transverse direction and the bond lines extend in the longitudinal direction, i.e., along the machine direction during manufacturing, and/or in the transverse direction, i.e., perpendicular to the machine direction. Such bond lines and in particular continuous bond lines along the machine direction simplify the manufacturing process. The regular pattern of mechanical fasteners may, e.g., also be a pattern consisting of staggered rows of mechanical fasteners.

The bond lines may be continuous or discontinuous lines. Thus, the bond lines may comprise interruptions. The bond lines may also comprise dots such that a number of dots form a bond line. The bond lines may also comprise combinations of continuous and/or discontinuous bond lines and/or dots.

The bond lines may be straight lines or curves or combinations thereof.

The present invention further relates to a method of manufacturing a web, preferably a web as described above. According to said method, a first web of a first material and a second web of a fastening material are provided. The second web has first and second major surfaces and is provided with a pattern of mechanical fasteners having stems and optionally caps on the first major surface. The method further comprises the step of bonding the second web to the first web with a second major surface of the second web facing the first web. The second web is bonded to the first web at regions between adjacent mechanical fasteners and the second web is not bonded to the first web at regions below the mechanical fasteners. The mechanical fasteners may optionally comprise an enlarged head portion such as caps to form hooks.

It is preferred that the step of bonding comprises welding, preferably ultrasound welding.

Preferably, the step of bonding is performed with a sonotrode having multiple horn tips arranged in a pattern adjusted to the pattern of mechanical fasteners. For example, the multiple horn tips of the sonotrode may be arranged in a pattern corresponding to free spots provided between the mechanical fasteners, i.e., a complementary pattern to the mechanical fastener pattern. For example, if the mechanical fasteners are arranged in lines, the sonotrode may have multiple horn tips arranged in lines between adjacent mechanical fastener lines. Alternatively, a line of horn tips may be arranged only every second or every third mechanical fastener line. If the pattern of mechanical fasteners comprises staggered rows of mechanical fasteners, the sonotrode could, e.g., have multiple horn tips arranged in a complementary pattern of staggered horn tip rows.

Preferably, the size of the horn tips is adjusted to the distance between adjacent mechanical fasteners. For example, the maximum diameter of the horn tips is preferably equal or less than 90%, more preferably equal or less than 80%, most preferably equal to or less than 70% of the distance between adjacent mechanical fasteners. Preferably, the multiple horn tips are arranged in a comb-like pattern and/or on a roll.

The step of bonding preferably comprises moving the first and second webs along the sonotrode. It is also preferred that the second web is guided along the sonotrode by means of interaction or engagement between the mechanical fasteners with the horn tips. For example, the stems of the mechanical fasteners may be permanently or intermittently in contact with one or more of the horn tips in order to guide the second web along the sonotrode. The horn tips may also have a T-shape or another shape complementary to the shape of the mechanical fasteners in order to better engage with the mechanical fasteners.

Preferably, the sonotrode further comprises an anvil comprising a surface structure adjusted to the pattern of mechanical fasteners. According to a preferred embodiment, the sonotrode and/or the anvil is a rotatable roll which is preferably adapted to transport the web and/or to guide the first and/or second layer through a nip formed between the sonotrode and the anvil.

The present invention further relates to a sonotrode and/or an anvil adapted to bond a fastening material to a non-woven material, wherein the sonotrode and/or the anvil comprise multiple horn tips arranged in a pattern. The multiple horn tips are preferably arranged in a comb-like pattern. Preferably, the multiple horn tips are arranged on a rotatable roll. The sonotrode and/or the anvil preferably further comprises an anvil comprising a surface structure adjusted to the pattern of the horn tips. Preferably, the horn tips have a width in the range between 0.09 mm and 0.49 mm, more preferably between 0.19 mm and 0.39 mm. The maximum width of the horn tips is determined by the distance between two adjacent hook rows. Preferably, the horn tips have a height in the range between 0.25 mm and 1.5 mm, more preferably between 0.45 mm and 1.3 mm. Preferably, the horn tips are trapezoidal and/or substantially T-shaped.

The above-described structures in the sonotrode may alternatively be formed in the anvil, while the sonotrode is not structured. This may reduce effort to manufacture such sonotrodes as these may be made of Titanium which is usually more difficult to form compared to anvils made from steel.

The sonotrode and/or the anvil may comprise surface structures on the anvil and/or the sonotrode such that continuous or discontinuous bond lines or dots may be formed. Alternatively, the sonotrode may be used such that the ultrasound energy or other welding energy such as laser, heat source etc., is periodically switched on and off to form discontinuous bond lines or dots.

The sonotrode and/or the anvil may also comprise horn tips shaped as needles or spikes in order to form discontinuous bond lines or dots.

The present invention further relates to the use of the sonotrode and/or the anvil described above for bonding a fastening material to a non-woven material. The mechanical fasteners of the fastening material are arranged in a pattern and the multiple horn tips of the sonotrode are arranged in a pattern corresponding to the pattern of mechanical fasteners.

The method and the sonotrode and/or the anvil according to the present invention are of use for a technically advanced manufacturing process of a web comprising a fastening material, which is bonded to a layer of another material. Once a suitable sonotrode has been built, the inventive method can be performed at high speeds, with high accuracy and at low costs. The inventive method results in the inventive web which has a superior web performance in terms of shear strength and peel strength compared to the traditional method of ultrasonic bonding independent of the hook rows, since no mechanical fasteners are being destroyed during the bonding process according to the present invention.

The following is a summary of embodiments of the invention:
1. A web comprising a first layer (1) of a first material and a second layer (2) of a fastening material, the second layer (2) having first and second major surfaces (3, 4) and being provided with mechanical fasteners (5) having stems (5a) and optionally caps (5b) on the first major surface (3), the second major surface (4) of the second layer (2) facing the first layer (1), wherein the second layer (2) is bonded to the first layer (1) at regions (6) between adjacent mechanical fasteners (5) and wherein the second layer (2) is not bonded to the first layer (1) at regions below the mechanical fasteners (5).
2. The web of embodiment 1, wherein the second layer (2) is bonded to the first layer (1) by welding.
3. The web of embodiment 2, wherein the second layer (2) is bonded to the first layer (1) by ultrasound welding or thermo bonding.
4. The web of any of the previous embodiments, wherein the first material is a non-woven.
5. The web of any of the previous embodiments, wherein the second layer (2) is provided with a regular pattern of mechanical fasteners (5) and wherein the second layer (2) is bonded to the first layer (1) along lines extending between adjacent mechanical fasteners (5).
6. The web of embodiment 5, wherein the web defines a longitudinal and a transverse direction and wherein the bond lines extend in the longitudinal and/or transverse direction.
7. The web of any of embodiments 1 to 6, wherein the bond lines are continuous or discontinuous lines or comprise dots or combinations thereof.
8. The web of any of embodiments 1 to 7, whereby the bond lines are straight lines or curved or combinations thereof.
9. A method of manufacturing a web, preferably a web according to any of the previous embodiments, the method comprising:
   providing a first web (1) of a first material;
   providing a second web (2) of a fastening material, the second web (2) having first and second major surfaces (3, 4) and being provided with a pattern of mechanical fasteners (5) having stems (5a) and optionally caps on the first major surface (3);
      wherein the second web (2) is bonded to the first web (1) at regions (6) between adjacent mechanical fasteners (5) and wherein the second web (2) is not bonded to the first web (1) at regions below the mechanical fasteners (5).
10. The method of embodiment 9, wherein the step of bonding comprises welding, preferably ultrasound welding.
11. The method of any of embodiments 9 or 10, wherein the step of bonding is performed with a sonotrode (10) and/or the anvil (12) having multiple horn tips (11) arranged in a pattern adjusted to the pattern of mechanical fasteners (5).
12. The method of embodiment 11, wherein the size of the horn tips (11) is adjusted to the distance between adjacent mechanical fasteners (5) and/or optionally stems (5a).
13. The method of any of embodiments 11 to 12, wherein the multiple horn tips (11) are arranged in a comb-like pattern and/or on a roll, and wherein the step of bonding comprises moving the first and second webs (1, 2) along the sonotrode (10).
14. The method of embodiment 13, wherein the second web (2) is guided along the sonotrode (10) by means of engagement between the mechanical fasteners (5) with the horn tips (11).
15. The method of any of embodiments 11 to 14, wherein the sonotrode (10) further comprises an anvil (12) comprising a surface structure adjusted to the pattern of mechanical fasteners (5).
16. The method of any of embodiments 9 to 15, wherein the first material is a non-woven.
17. A sonotrode (10) and/or an anvil (12) adapted to bond a fastening material to a non-woven material, the sonotrode (10) comprising multiple horn tips (11) arranged in a pattern.
18. The sonotrode and/or an anvil (12) of embodiment 17, wherein the multiple horn tips (11) are arranged in a comb-like pattern.
19. The sonotrode and/or an anvil (12) of embodiment 17, wherein the multiple horn tips (11) are arranged on a rotatable roll.
20. The sonotrode and/or an anvil (12) of any of embodiments 17 to 19, further comprising an anvil (12) and/or a sonotrode (10) comprising a surface structure adjusted to the pattern of the horn tips (11).
21. The sonotrode of any of embodiments 17 to 20, wherein the horn tips (11) have a width in the range between 0.09 mm and 0.49 mm, preferably between 0.19 mm and 0.39 mm.
22. The sonotrode of any of embodiments 17 to 21, wherein the horn tips (11) have a height in the range between 0,25 and 1,5, preferably between 0,45 and 1,3.
23. The sonotrode and/or an anvil (12) of any of embodiments 17 to 22, wherein the horn tips (11) are trapezoid and/or substantially T-shaped.
24. Use of the sonotrode (10) and/or an anvil (12) of any of embodiments 17 to 23 for bonding a fastening material to a non-woven material, wherein the mechanical fasteners (5) of the fastening material are arranged in a pattern and wherein the multiple horn tips (11) of the sonotrode (10) are arranged in a pattern corresponding to the pattern of mechanical fasteners (5).

Preferred embodiments of the present invention are further illustrated with reference to the following Figures.
- Fig. 1:: shows a photograph of a fastening tab for a diaper according to the prior art.
- Fig. 2:: shows a microphotograph of the fastening material comprising hooks of the fastener shown in Fig. 1.
- Fig. 3:: shows a magnified section of the microphotograph shown in Fig. 2.
- Fig. 4:: shows a perspective view of a sonotrode according to the present invention.
- Fig. 5:: shows a schematic cross-section through a sonotrode according to the present invention.
- Fig. 6:: shows a magnified section of Fig. 5.
- Fig. 7:: shows a photograph of a web according to the present invention.
- Fig. 8:: shows a cross-section through a web according to the present invention.
- Figs. 9 - 12:: show schematic perspective views of a sonotrode according to the present invention.

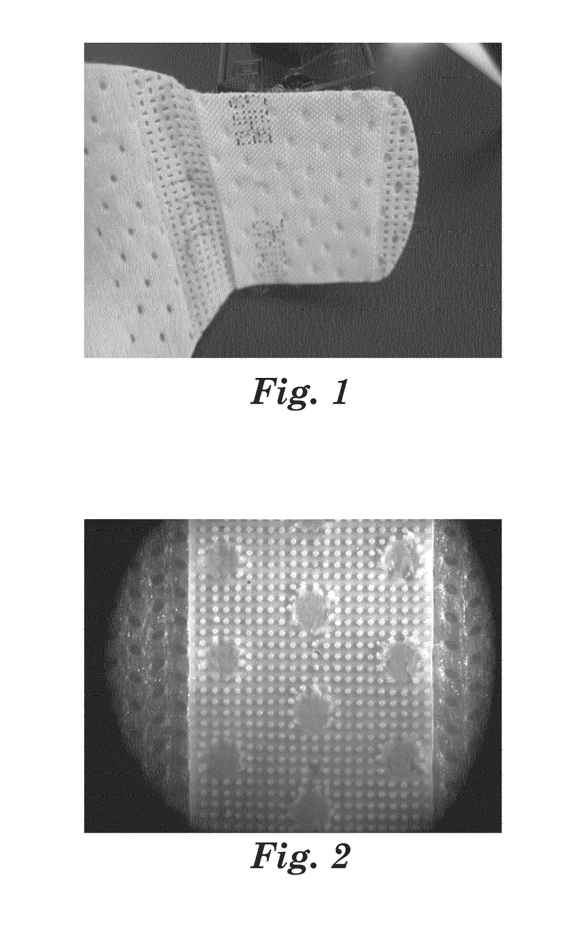

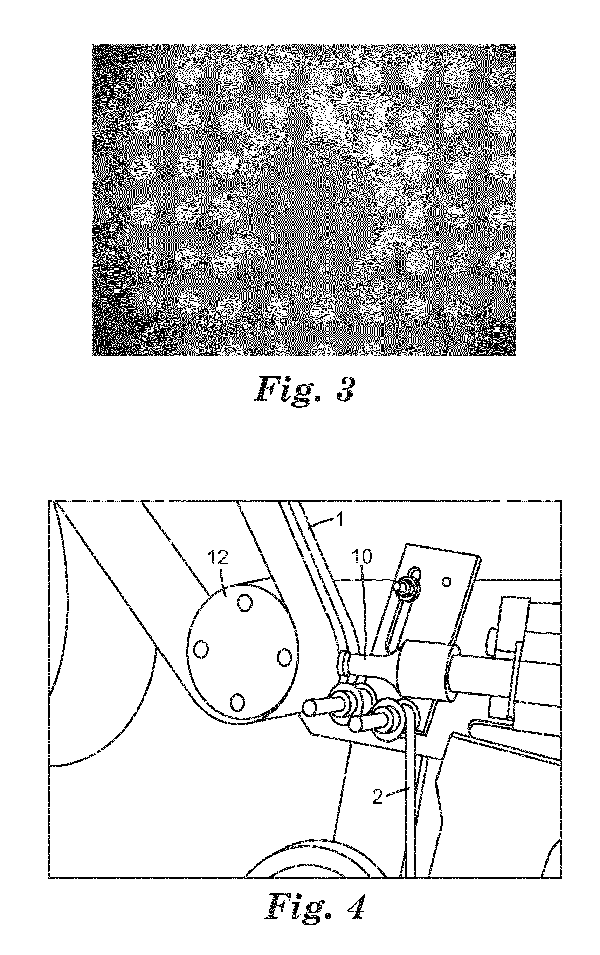

Fig. 1 shows a photograph of a prior art fastening tap for a diaper. The fastening tab comprises a web consisting of a non-woven layer and a hook material layer which are bonded to each other by means of ultrasound welding at specific bonding spots which are separate from each other and which are visible in Fig. 1 as "indentations" in the hook material. These indentations are better recognizable in the magnified photographs of Figs. 2 and 3. As is evident from those Figures, the bonding spots of these prior art webs cover an area of several hooks (about nine hooks in Fig. 3) and the hooks which had been present at the bonding spots previously are completely destroyed due to the bonding by ultrasound welding, which basically melts the hook material in the entire bonding spot. For example, in the magnified bonding spot visible in Fig. 3 seven hooks have been entirely erased and at least five further hooks are substantially damaged. These bonding spots of the prior art web thus detrimentally affect the performance of the material, e.g., shear and peel strength, since less (intact) hooks are available for engagement with a loop material.

In order to overcome this problem the present invention provides a web comprising a first layer of a first material and a second layer of a fastening, e.g. hook, material with the second layer being bonded to the first layer at regions between adjacent hook stems only. This may, e.g., be achieved by means of a sonotrode comprising multiple horn tips arranged in a pattern. An example of a manufacturing device comprising such a sonotrode 10, an anvil 12 for bonding a first web 1 to a second web 2 is shown in Fig. 4.

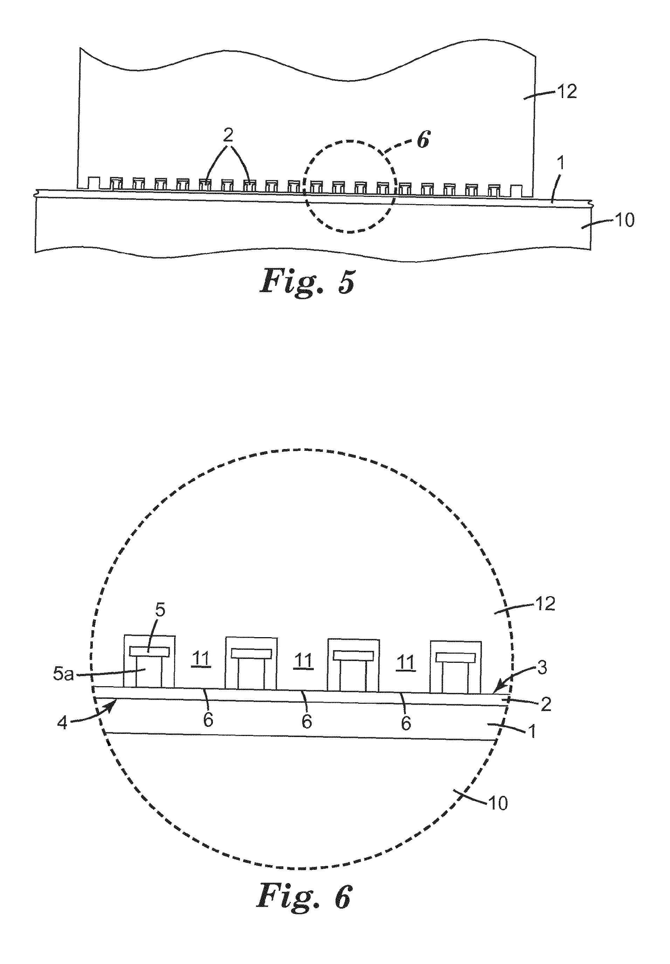

According to the inventive method, a first web 1 of a first material such as, e.g., a non-woven is provided and a second web 2 of a fastening material such as, e.g., a hook material having first and second major surfaces 3 and 4 is provided with a pattern of hooks 5 having stems 5a on the first major surface 3 (see Fig. 6). The second web 2 is bonded to the first web 1 with the second major surface 4 of the second web 2 facing the first web 1, wherein the second web 2 is bonded to the first web 1 at regions 6 between adjacent hook stems 5a.

According to a preferred embodiment, the step of bonding is performed with a sonotrode 10 having multiple horn tips 11 arranged in a pattern adjusted to the pattern of hooks 5. As shown in Fig. 6, the size of the horn tips 11 is adjusted to the distance between adjacent hooks 5 and the multiple horn tips 11 are preferably arranged in a comb-like pattern, wherein the first and second webs 1 and 2 are moved along the sonotrode 10, preferably between the sonotrode 10 and an anvil 12.

Since the specific sonotrode having the multiple horn tips allows a well defined energy transfer to the bonding regions 6 only, the first and second webs 1 and 2 are bonded only at these regions 6 and the hooks 5 and the stems 5a are not detrimentally affected by the welding process. As shown in Fig. 4 the first and second webs 1 and 2 may be continuously fed through a nip between the sonotrode 10 and the anvil 12. Of course, the dimensions shown in Fig. 6 are exemplary only and the horn tips 11 of the sonotrode 10 may have an entirely different shape and/or dimension as long as the shape and/or dimension of the horn tips 11 is adjusted to the distance between adjacent hooks and/or hook stems and/or the shape of the hooks. For example, the horn tips 11 of the sonotrode 10 could also have a T-shaped profile in order to better engage the interstices between adjacent hooks 5.

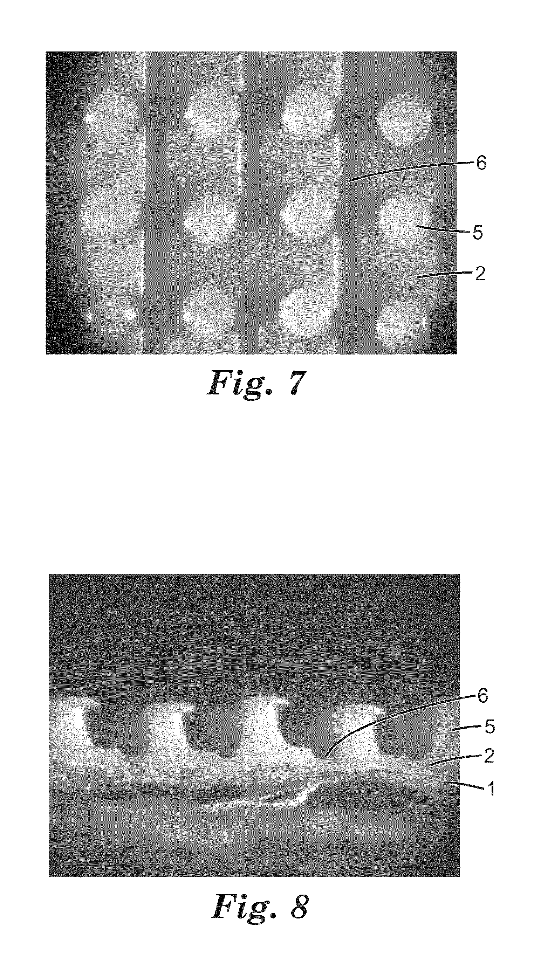

The resulting web according to the present invention is shown in the (micro-)photographs of Figs. 7 and 8 showing the inventive web in a top view (Fig. 7) and a cross-sectional view (Fig. 8). As may be taken from these Figures and is most evident from Fig. 7, the inventive web comprises bond lines 6 which extend along the machine direction between the hook rows. The hooks 5 are arranged in Fig. 7 in a square array. The distance between two adjacent hook rows is 0.27 mm (hook cap to hook cap distance). The bond lines are continuous in the machine direction and arranged between every hook row. The second web 2 of hook material is bonded to the first web underneath said hook material along these bond lines 6 only. These bond lines 6 extend between adjacent lines of the hooks 5. While Fig. 7 shows the bond lines 6 as having a slight bias to the left hand side it is preferred that the bond lines 6 are substantially centered with respect to adjacent lines of hooks 5.

As may be taken from the cross-section shown in Fig. 8, the second layer 2 of hook material is only affected by the bonding step at the bonding regions or lines 6. The surrounding hook material and in particular the hooks, however, remain intact.

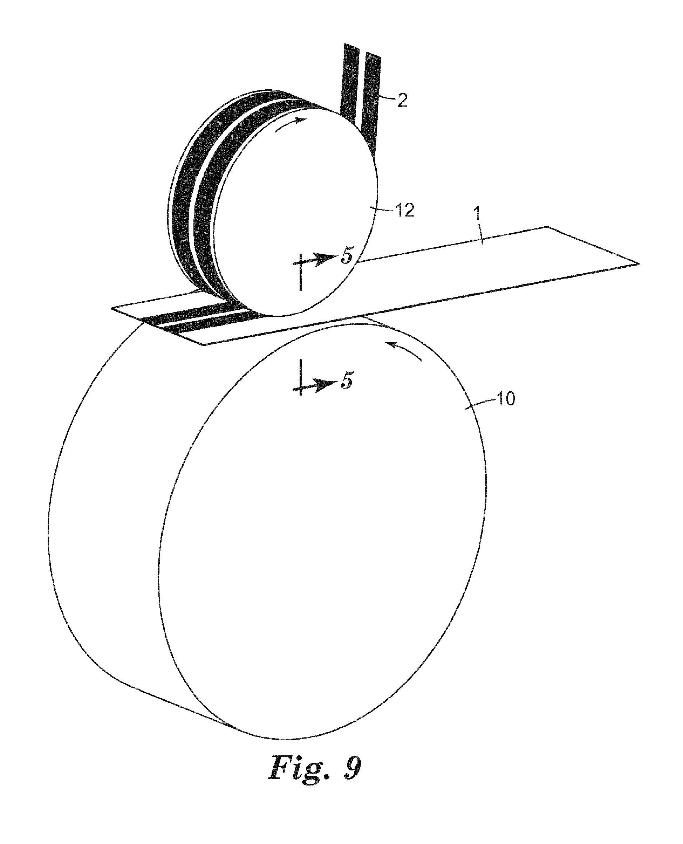

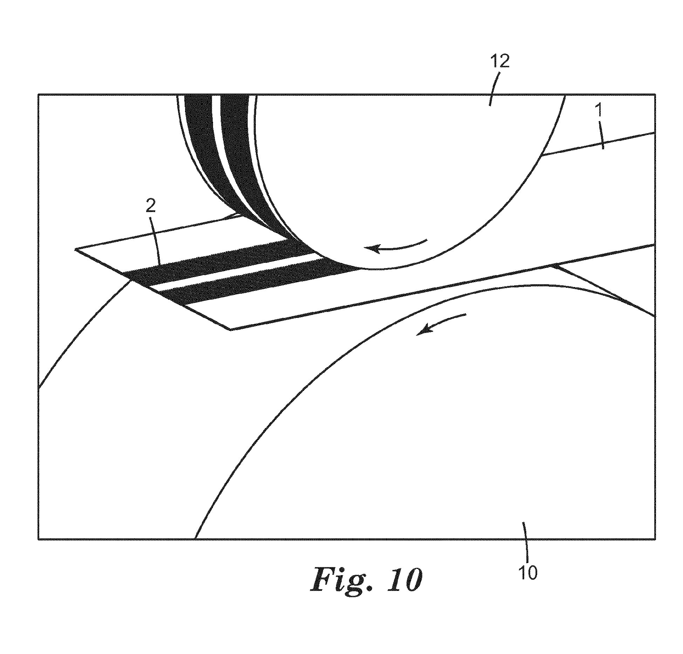

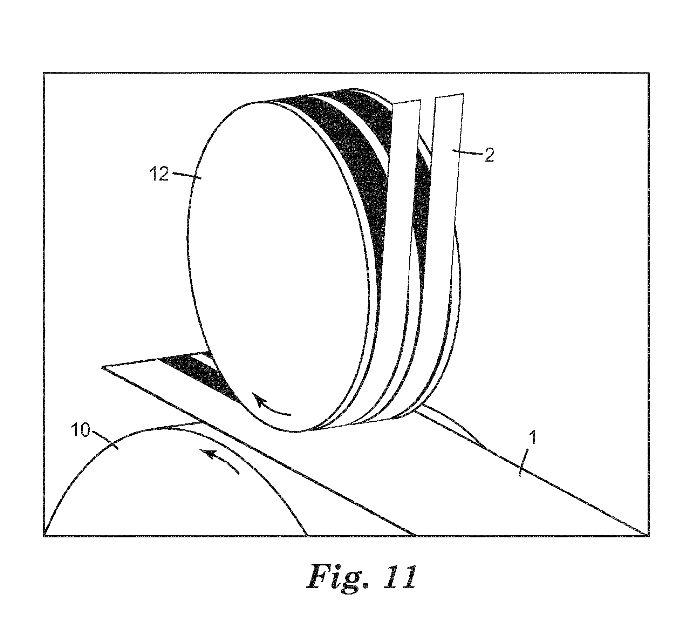

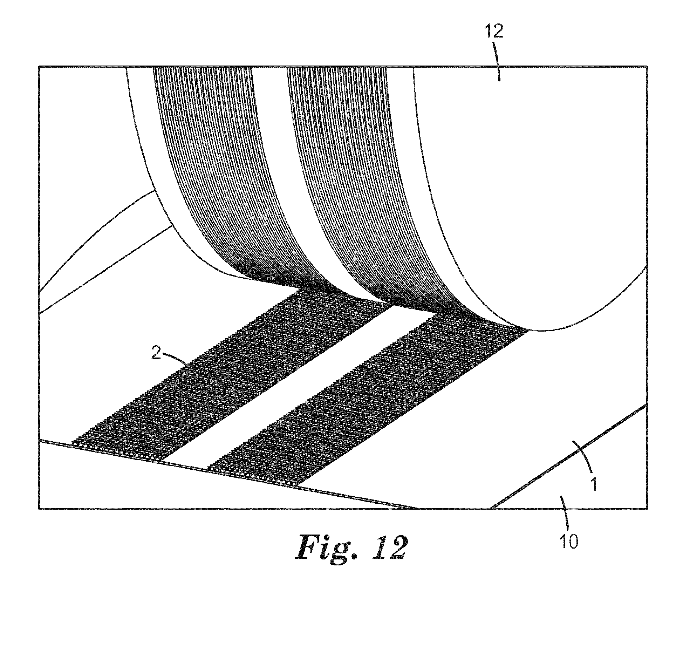

The preferred embodiment of the sonotrode according to the present invention is shown in the schematic perspective views of Figs. 9 - 12. The device for manufacturing the inventive web shown in these Figures comprises a rotatable sonotrode roll 10 and a rotatable anvil roll 12. The two rolls form a nip to which a first web 1 of a first material and a second web 2 of a hook material are fed by rotating the two rolls in order to bond the second web of hook material to the first web at regions between adjacent hooks stems only. As can be seen in Fig. 12, the anvil roll 12 may comprise multiple horn tips arranged in a pattern. In the example shown, the anvil roll 12 comprises multiple horn tips arranged in circumferential lines whose spacing is adjusted to the spacing of the hooks in the second web 2 of hook material. Moreover, the sonotrode roll 10 may, as shown in Fig. 12, also comprise a surface adjusted to the pattern of the horn tips. In the example shown in Fig. 12, the anvil comprises a pattern of rectangular protrusions. Said pattern helps to focus the ultrasonic energy provided by the sonotrode to the bonding regions only.

In order to compare the performance of the hooks of a prior art web with the hooks of a web according to the present invention four different samples have been prepared. The hook material for the second layer was either the microreplicated hook CHK-00732 (available from 3M) or the material designated as M2. The hooks of the CHK-00732 material were made of polypropylene and the basis weight of the second layer was 117 g/m² at a thickness of 100 µm The basis weight of the M2 material (which was similar to CHK-00732) was 180 g/m² at a thickness of 170 µm. Both materials had roughly 1,600 hooks of a diameter of about 0.35 mm per square inch. The hook material for the second layer was bonded by means of ultrasound welding to a non-woven (Pegatex S, a spunbond made from polypropylene having a density of 70 g/m², available from Pegas). Bonding was performed according to the present invention as shown in Figs. 7-11 (Examples 1 and 2) as well as according to the prior art technique as shown in Figs. 1 and 2 (Comparative Examples 1 and 2).

Welding on all Examples and Comparative Examples was performed with simple test equipment shown in Fig. 4. The ultrasonic generator used was from Telsonic Ultrasonics, Bronschhofen, Switzerland, had a power of 500 W and worked with an operation frequency of 36 kHz. The weld speed was 1 m/min. For the Examples, the sonotrode surface had horn tips and the anvil was flat.

For the Comparative Examples, the pattern of the anvil was 6 mm x 10 mm and each pin had a diameter of 0.2 mm and the sonotrode was flat. The anvil was made of aluminum and the pins were made of carbide metal. The sonotrode was made of titanium. For the Examples, a special milled sonotrode was made of titanium. The milling tool was a 0.4 mm wide saw blade. The distance between each row was 0.62 mm suitable for the M2 hook material and CHK-0732 hook material. The height of the horn tips was 0.7 mm. The anvil was made of aluminum and had a flat surface.

A peel strength test according to the standard test method ASTM D5170-98 and a shear strength test according to the standard test method ASTM D5169-98 were performed on each of these Examples and Comparative Examples (using a hook width of 13 mm and a sample length of 25 mm) using either EBL Light (available from 3M), a nonwoven landing zone made of corrugated polypropylene fibers bonded to an extruded film, or a knitted loop material (aplix N31) as a landing zone.

The results of the peel strength test using ten different samples for each range are shown in the following table for different ranges (The results of the peel strength test for 0-20 mm, 18-24 mm and up to 40 mm):

| RANGE | HOOK | TYPE OF WELDING | EBL-light [N] | Knitted loop [N] |
|---|---|---|---|---|
| 0-6mm | CHK-0732 | Welded between hook pins | 3,14 | 2,78 |
| 0-6mm | CHK-0732 | Pattern welding | 2,29 | 2,29 |
| 0-6mm | M2 hook | Welded between hook pins | 2,69 | 4,49 |
| 0-6mm | M2 hook | Pattern welding | 2,07 | 3,16 |
| 6-12mm | CHK-0732 | Welded between hook pins | 8,3 | 7,73 |
| 6-12mm | CHK-0732 | Pattern welding | 5,91 | 6,85 |
| 6-12mm | M2 hook | Welded between hook pins | 7,23 | 6,96 |
| 6-12mm | M2 hook | Pattern welding | 5,86 | 7,84 |
| 12-18mm | CHK-0732 | Welded between hook pins | 11,13 | 11,99 |
| 12-18mm | CHK-0732 | Pattern welding | 7,15 | 9,7 |
| 12-18mm | M2 hook | Welded between hook pins | 10,03 | 12,78 |
| 12-18mm | M2 hook | Pattern welding | 8,47 | 9,38 |
| 18-24mm | CHK-0732 | Welded between hook pins | 11,44 | 14,2 |
| 18-24mm | CHK-0732 | Pattern welding | 8,83 | 12,09 |
| 18-24mm | M2 hook | Welded between hook pins | 13,37 | 15,66 |
| 18-24mm | M2 hook | Pattern welding | 9,7 | 13,72 |
| 30-end | CHK-0732 | Welded between hook pins | 10,22 | 16,49 |
| 30-end | CHK-0732 | Pattern welding | 7,99 | 14,06 |
| 30-end | M2 hook | Welded between hook pins | 5,43 | 15,02 |
| 30-end | M2 hook | Pattern welding | 3,17 | 10,13 |
| 0-20mm | CHK-0732 | Welded between hook pins | 11,56 | 12,93 |
| 0-20mm | CHK-0732 | Pattern welding | 7,82 | 10,06 |
| 0-20mm | M2 hook | Welded between hook pins | 11,77 | 13,97 |
| 0-20mm | M2 hook | Pattern welding | 9,24 | 10,28 |
| Fmax | CHK-0732 | Welded between hook pins | 12,8 | 16,95 |
| Fmax | CHK-0732 | Pattern welding | 10,2 | 14,87 |
| Fmax | M2 hook | Welded between hook pins | 13,74 | 16,85 |
| Fmax | M2 hook | Pattern welding | 10,13 | 14,46 |

The results of the shear strength test using ten different samples for each (Comparative) Example are shown in the following table:

| RANGE | HOOK | TYPE OF WELDING | EBL-light | Knitted loop |
|---|---|---|---|---|
| F-max | CHK-0732 | Welded between hook pins | 56,1 | 48,66 |
| F-max | CHK-0732 | Pattern welding | 49,6 | 45,4 |
| F-max | M2 hook | Welded between hook pins | 55,3 | 48,63 |
| F-max | M2 hook | Pattern welding | 48,45 | 43,27 |

As one may take from these results, with any landing zone material and with both different hook samples the webs according to the present invention show better peel and shear performance than the prior art webs.

## Claims

1. A web comprising a first layer (1) of a first material and a second layer (2) of a fastening material, the second layer (2) having first and second major surfaces (3, 4) and being provided with mechanical fasteners (5) having stems (5a) and optionally caps (5b) on the first major surface (3), the second major surface (4) of the second layer (2) facing the first layer (1), wherein the second layer (2) is bonded to the first layer (1) at regions (6) between adjacent mechanical fasteners (5) and wherein the second layer (2) is not bonded to the first layer (1) at regions below the mechanical fasteners (5).

2. The web of claim 1, wherein the second layer (2) is bonded to the first layer (1) by welding, preferably ultrasonic welding, or thermo bonding.

3. The web of any of the previous claims, wherein the first material is a non-woven.

4. The web of any of the previous claims, wherein the second layer (2) is provided with a regular pattern of mechanical fasteners (5) and wherein the second layer (2) is bonded to the first layer (1) along lines extending between adjacent mechanical fasteners (5).

5. The web of claim 4, wherein the web defines a longitudinal and a transverse direction and wherein the bond lines extend in the longitudinal and/or transverse direction.

6. The web of any of claims 1 to 5, wherein the bond lines are continuous or discontinuous lines or comprise dots or combinations thereof.

7. The web of any of claims 1 to 6, whereby the bond lines are straight lines or curved or combinations thereof.

8. A method of manufacturing a web, preferably a web according to any of the previous claims, the method comprising:
providing a first web (1) of a first material;
providing a second web (2) of a fastening material, the second web (2) having first and second major surfaces (3, 4) and being provided with a pattern of mechanical fasteners (5) having stems (5a) and optionally caps on the first major surface (3);
wherein the second web (2) is bonded to the first web (1) at regions (6) between adjacent mechanical fasteners (5) and wherein the second web (2) is not bonded to the first web (1) at regions below the mechanical fasteners (5).

9. The method of claim 8, wherein the step of bonding comprises welding, preferably ultrasound welding, or thermo bonding.

10. The method of any of claims 8 or 9, wherein the step of bonding is performed with a sonotrode (10) and/or an anvil (12) having multiple horn tips (11) arranged in a pattern adjusted to the pattern of mechanical fasteners (5).

11. The method of claim 10, wherein the size of the horn tips (11) is adjusted to the distance between adjacent mechanical fasteners (5) and/or stems (5a).

12. The method of any of claims 10 or 11, wherein the multiple horn tips (11) are arranged in a comb-like pattern and/or on a roll, and wherein the step of bonding comprises moving the first and second webs (1, 2) along the sonotrode (10).

13. The method of claim 12, wherein the second web (2) is guided along the sonotrode (10) by means of engagement between the mechanical fasteners (5) with the horn tips (11).

14. The method of any of claims 10 to 13, wherein the sonotrode (10) further comprises an anvil (12) comprising a surface structure adjusted to the pattern of mechanical fasteners (5).

15. The method of any of claims 8 to 14, wherein the first material is a non-woven.

16. A sonotrode (10) and/or an anvil (12) adapted to bond a fastening material to a non-woven material, the sonotrode (10) comprising multiple horn tips (11) arranged in a pattern, wherein the multiple horn tips (11) are arranged in a comb-like pattern and/or wherein the multiple horn tips (11) are arranged on a rotatable roll, and wherein the horn tips (11) have a width in the range between 0.09 mm and 0.49 mm, preferably between 0.19 mm and 0.39 mm and wherein the horn tips (11) have a height in the range between 0.25 and 1.5 mm, preferably between 0.45 and 1.3 mm.

17. The sonotrode and/or the anvil (12) of claim 16, further comprising a surface structure on the anvil (12) and/or on the sonotrode (10) adjusted to the pattern of the horn tips (11).

18. Use of the sonotrode (10) and/or the anvil (12) of any of claims 16 or 17 for bonding a fastening material to a non-woven material, wherein the mechanical fasteners (5) of the fastening material are arranged in a pattern and wherein the multiple horn tips (11) of the sonotrode (10) are arranged in a pattern corresponding to the pattern of mechanical fasteners (5).
